# EUROPÄISCHE PATENTANMELDUNG

(11) **EP 1 970 046 A1**
(43) Veröffentlichungstag der Anmeldung: **17.09.2008**
(21) Anmeldenummer: 08004451.4
(22) Anmeldetag: 11.03.2008
(51) Int. Cl.: A61K 8/49, A61Q 5/12, A61Q 7/00

(54) **Verwendung von Purin und Purinderivate zur Haarbehandlung**

(30) Priorität: 15.03.2007 DE 102007013143
(71) Anmelder: Henkel AG & Co. KGaA, 40589 Düsseldorf (DE)
(72) Erfinder: Welß, Thomas, 40591 Düsseldorf (DE); Giesen, Melanie, 47608 Geldern (DE); Ghosh, Robin, 40215 Düsseldorf (DE); Briese, Melanie, 40597 Düsseldorf (DE); Özkabakcioglu, Yasmin, 40225 Düsseldorf (DE)

(57) **Zusammenfassung**

Die vorliegende Erfindung betrifft die Verwendung von Purin und/oder Purinderivat(en) in kosmetischen Mitteln zur Fiegulation der Haarkeratine und/oder Verhinderung von Altershaar, insbesondere in Haarbehandlungsmitteln, wie Shampoos, Haamachspülmitteln, Haargele, Haartonics, Haarwasser, Haarkuren, Haarcreme, Haarlotionen, Haarsprays und Haartinkluren.

## Beschreibung

Die vorliegende Erfindung betrifft die Verwendung von Purin und/oder Purinderivat(en) in kosmetischen Mitteln zur Regulation der Haarkeratine und/oder Verhinderung (Verminderung) von Altershaar,

Die menschliche Haut mit Ihren Anhangsgebilden ist ein sehr komplex aufgebautes Organ, welches aus einer Vielzahl verschiedener Zelltypen besteht. Jede lebende Zelle dieses Organs ist in der Lage auf Signale ihrer inneren und äußeren Umwelt, zu reagieren. Diese Reaktionen der Zellen werden durch eine geordnete Regulation auf Gen- und Proteinebene realisiert, so dass der Metabolismus von Zellen der Haut und Ihrer Anhangsgebilde nicht statisch sondern sehr dynamisch ist. Die Reaktionen der Haut und/oder ihrer Anhangsgebilde auf Veränderungen der Umgebung dürfen jedoch nicht als Reaktionen einzelner, isolierter Zellen betrachtet werden. Vielmehr ist jede Zelle in ein komplexes Kommunikationsnetzwerk eingebunden. Dieses Netzwerk beinhaltet z.B. die Kommunikation zwischen Zellen der Epidermis und Zellen der Dermis. An der Kommunikation zwischen den Zellen der Haut und/oder ihrer Anhangsgebilde sind Signalmoleküle wie z.B. Interleukine, Wachstumsfaktoren (z.B. KGF, EGF oder FGF) usw. beteiligt.

Die Menge eines haaraktiven Wirkstoffes, der üblicherweise transdermal und speziell transfollikulär bis zum Haarbulbus penetrieren kann, ist äußerst gering und hängt im Wesentlichen von den physikochemischen Eigenschaften der Substanz selber (z.B: Größe, Ladung, Lipophilie) sowie der Wahl der Formulierung ab.

Haarfollikelzellen unterliegen einem genetisch festgelegten Zyklus von Wachstum, Regression, und Ruhephase. Der Haarfollikel ist damit das einzige Organ, dass sich ständig selbst erneuert und somit einen, in Abhängigkeit von der jeweiligen Wachstumsphase, einzigartigen Metabolismus aufweist. So kommt der Metabolismus des Haarfollikels in der Ruhephase fast völlig zum Erliegen und wird mit jedem neuem Beginn eines weiteren Zyklus ebenfalls neu Initiiert.

Gesteuert wird dieser Zyklus von einer kleinen, hochspezialisierten Zellpopulation im Haarbulbus, den dermalen Papillenzellen, die durch ein komplexes Set molekularer Signale, das spezifisch für jede Phase des Haarzyklus ist, das Haerwachstum kontrollieren (Botchkarev VA et al. (2003) J Investig Dermatol Symp Proc 8:46-5).

Je nach Anwendungsgebiet unterscheidet man ein breite Palette kosmetischer Mittel, beispielsweise zur Hautpflege (Badepräparate, Hautwasch- und -reinigungsmittel, Hautpflegemittel, Augenkosmetika, Lippenpflegemittel, Nagelpflegemittel, Intimpflegemittel, Fußpflegemittel), solche mit spezieller Wirkung (Lichtschutzmittel, Hautbräunungsmittel, Depigmentierungsmittel, Desodorantien, Antihidrotika, Haarentfernungsmittel, Rasiermittel, Duftmittel) und solche zur Haarpflege (Haarwaschmittel, Haarpflegemittel, Haarverfestigungsmittel, Haarverformungsmittel, Mittel zur Farbänderung).

Kosmetische Mittel im Sinne der vorliegenden Erfindung sind daher beispielsweise ausgewählt aus der Gruppe der Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen.

Bei den kosmetischen Mitteln besteht ein Bedarf nach Inhaltsstoffen, die den Zubereitungen weiteren Nutzen verleihen, sei es aus anwendungstechnischer Sicht, aus herstelltechnischer Sicht oder aus Sicht des Verbrauchers, der beispielsweise aufgrund bestimmter Inhaltstoffe ein verbessertes subjektives Empfinden dem kosmetischen Mittel gegenüber entwickelt. Der Schwerpunkt der Leistung eines Inhaltsstoffes kann dabei je nach kosmetischem Mittel auch unterschiedlich sein. So kann ein und derselbe Stoff in einem Shampoo oder einer Spülung pflegende Eigenschaften für die behandelten Haare bewirken, während er in einem Haarfärbemittel darüber hinaus die Anfärbung der Kopfhaut verringert oder die zum Teil aggressive Wirkung des Färbemittels kompensiert.

Es besteht daher auf dem Gebiet der Kosmetik ständig der Wunsch, neue Einsatzstoffe bereitzustellen, die in einzelnen, vorzugsweise in vielen Produktkategorien vorteilhafte Wirkungen entfallen.

Es wurde nun gefunden, daß Purin und/oder Purinderivat(e) mit besonderem Vorzug in kosmetische Mittel einarbeiten lassen und diesen Mitteln sowie den mit diesen Mitteln behandelten Teilen des Körpers, beispielsweise den Haaren, durch die Regulation der Haarkeratine, vorteilhafte Eigenschaften verleihen. Ebenfalls wurde gefunden, dass die Bildung von Altershaar verhindert bzw. vermindert werden konnte. Besonders ausgeprägt sind diese Effekte auf dem Haar (Kopfhaut), so daß Purin und/oder Purinderivat(e) erfindungsgemäß bevorzugt in kosmetische Mittel wie Haarbehandlungsmittel verwendet wird.

Gegenstand der vorliegenden Erfindung ist daher die Verwendung von Purin und/oder Purinderivat(en) in kosmetischen Mitteln zur Regulation der Haarkeratine und/oder Verhinderung (bzw. Verminderung) von Altershaar.

Im Laufe der chronologischen Alterung entsteht eine fortschreitende Verringerung der Haardichte (Haare/cm²) sowie des Haardurchmessers.

Weiter wurden altersbedingte funktionale Veränderungen im Haarfollikel gefunden, die zu einer verminderten Selbsterneuerungskapazität der Haarwurzel führt. Dieses Potential zur Selbsterneuerung ist die Grundlage des Haarzykluses eines gesunden Haares, der von sich abwechselnden Phasen des Wachstum, der Regression und der Ruhe geprägt ist.

Im Laufe der Haaralterung erfolgen eine verringerte Wachstums- und Zellteilungsrate sowie eine verkürzte Wachstumsphase. Dies führt häufig unter anderem zu feinerem und brüchigerem Haar. Darüber verlängert sich das Intervall zwischen zwei Haarzyklen.

Die verringerte Aktivität des Haarfollikels wird von der Abnahme verschiedener metabolischer Prozesse begleitet. So sinkt beispielsweise die Aktivität bestimmter Enzyme, die für die Zellteilung, den Energiestoffwechsel sowie die Detoxifizierung verantwortlich sind. Molekularbiologische Untersuchungen haben ergeben, dass neben verschiedenen Wachstumsfaktoren vor allem Intermediärfilamente wie Haarkeratine und keratinassoziierte Proteine (KAPs) altersbedingten Regulationen unterliegen.

Die Festigkeit der Haare und die Haarstruktur ist im Wesentlichen von der Zusammensetzung spezieller haarspezifischer Strukturproteine abhängig, den Haarkeratinen und den keratinassoziierten Proteinen. Durch die altersbedingte Veränderung der Zusammensetzung dieser spezifischen Proteine wird auf biologischer Ebene Einfluss auf die Haarstruktur genommen. So nimmt die Expression der Strukturproteine mit zunehmendem Alter ab, was häufig zu dünnerem und brüchigerem Haar führt.

Des Weiteren erhöht sich in älteren Haarfollikeln die Aktivität von Caspasen, die eine zentrale Rolle bei der Steuerung apoptotischer Prozesse spielen. Unter Apoptose versteht man den programmierten Zelltod. Er manifestiert sich zunächst in morphologischen Veränderungen wie dem Schrumpfen der Zellen und Ausbildung von Membreneinstülpungen, weiterhin zeigt sich eine Fragmentierung der chromosomalen DNA und es kommt letztlich zum Absterben und Abbau der betroffenen Zellen.

Die so untersuchten zellpysiologischen Veränderungen nehmen dabei Einfluss auf den Haarzyklus, das Wachstum und die Struktur der Haarfollikel und sind somit ursächlich für die phänotypischen Veränderungen der Haare im Alter.

Die systematische Identifizierung der im Alter modifizierten zellulären Prozesse ermöglicht die gezielte Entwicklung biologisch aktiver Haarpflegeprodukte, die genau auf die Bedürfnisse des älteren Haarfollikels abgestimmt sind und so den Zeichen der Follikelalterung entgegenwirken. So können die altersbedingten Veränderungen im Haarfollikel kompensiert bzw. vermindert werden.

Daher wird hiermit im Rahmen der vorliegenden Erfindung unter Verminderung bzw. Verhinderung von Altershaar die Kompensierung/Verminderung der altersbedingten Veränderungen im Haarfollikel verstanden.

Unter Altershaar im Sinne der Erfindung wird insbesondere Haar von Personen in einem Alter von über 50 Jahren verstanden sowie solches Haar, welches die vorstehend genannten Probleme aufweist.

Als Auswirkungen des Alterungsprozesses auf das Haar sind insbesondere eine Schwächung der Struktur, Dünner und/oder Brüchiger Werden des Haares, eine Verminderung der Expression von Wachstumsfaktoren, eine Reduktion apoptotischer Prozesse sowie eine Verminderung der Produktion von Strukturproteinen umfasst.

Die Verminderung bzw. Verhinderung des Altershaars im Sinne der vorliegenden Erfindung bedeutet daher insbesondere eine Stärkung des Haars, eine Verdickung des Haares, die Förderung der Expression von Haarkeratinen, Strukturverbesserung und/oder eine Verminderung bzw. Verhinderung von brüchig werden bzw. dünner werden des Haars.

Die VerhinderungNerminderung von Haarausfall, die Stimulation bzw. Beschleunigung des Haarwuchses ist von diesem Begriff erfindungsgemäß nicht umfasst.

Vorzugsweise werden Purin und/oder Purinderivat(e) der allgemeinen Formel (I) verwendet: 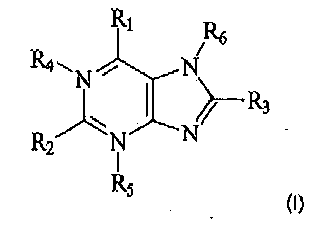 in der die Reste R¹, R² und R³ unabhängig voneinander ausgewählt sind aus -H, - OH, -NH₂, -SH und die Reste R⁴, R⁵ und K⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ =H)
- Adenin (R¹ =NH₂, R² = R³ R⁴ = R⁵ R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = H)
- Theophyllin (R¹ = R² = OH, R³ = H, R⁴ = CH₃, R⁵ = CH₃, R⁶ = H)

Bevorzugt enthalten die kosmetischen Mittel 0,0005 bis 5 Gew.-% Purin und/oder Purinderivat(e).
In einer weiteren bevorzugten Ausführungsform enthalten die kosmetischen Mittel 0,001 bis 2,5 Gew.-%, vorzugsweise 0,0025 bis 1 Gew.-%, besonders bevorzugt 0,005 bis 0,5 Gew.-% und insbesondere bevorzugt 0,01 bis 0,1 Gew.-% Purin(e) und/oder Purinderivat(e).
Vorzugsweise ist das Prurinderivat Theophyllin.

Vorzugsweise enthalten die erfindungsgemäß verwendeten Mittel zusätzlich mindestens einen Emulgator bzw. ein Tensid, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind.

In Abhängigkeit von der Art des erfindungsgemäß verwendeten Mittels kann es erforderlich sein, dass diese unterschiedliche Tenside enthalten. Dies gilt Insbesondere für Shampoos. Aber auch andere Mittel, wie beispielsweise Haarspülungen, Haarkuren, Haarwasser, Haarcreme, Haarlotionen, Haartinkturen.

Erfindungsgemäß können die verwendeten Mittel daher beispielsweise kationische Tenside ausgewählt aus quartären Ammoniumverbindungen, Esterquats und Amidoaminen enthalten.

Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersatze von Fettsäuren mit Triethanolamin, quaternierte Estersalze von Fettsäuren mit Diethenolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

In einer bevorzugten Ausführungsform der vorliegenden Erfindung wird, beispielsweise in einem Shampoo, eine Kombination aus anionischen und nichtionischen Tensiden oder eine Kombination aus anionischen und amphoteren Tensiden eingesetzt. In einem Haartonic kann der Fachmann jedoch auch weitgehend oder vollständig auf den Einsatz von Tensiden verzichten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen In der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulforlate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist.
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkonyletherphosphate der Formel (E1-I), 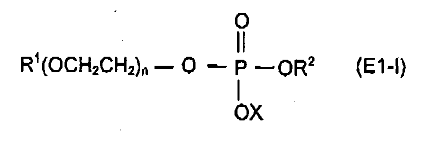 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III) 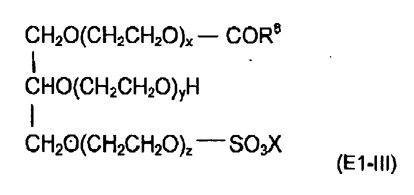 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sullate sind die Umsetzungsprodukte von Laurinräuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze, Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteilsäuremonoalkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die Im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammenium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidezoline mit jeweils 8 bis 18 C-Atomen in derAlkyl-oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe Im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomerl in der Alkylgruppe, wie beispielsweise die unter den Verkäufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₆-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Leurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoteylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 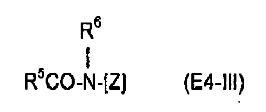 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Feltsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁶-CH₂-(CHOH)₄CH₂OH (E4-IV)

Vorzugsweise werden als Fettsäure-N-alkylpolyhydroxyalkylamlde Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Leuryl, 1-Myristyl, 1-Cetyl und 1-Stearyl. Besonders bevorzugt sind 1-Octyl, 1-Decyl, 1-Lauryl, 1-Myristyl, Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäß verwendete Mittel erfüllt. Handelt es sich um ein Shampoo oder ein anderes reinigendes Mittel, sind auch Tensidmengen über 45 Gew.-% üblich.

Gemäß einer weiteren, bevorzugten Ausführungsform der Erfindung enthalten die Mittel weiterhin mindestens einen Emulgator und/oder mindestens einen Dialkylether. Durch den Zusatz einer oder mehrerer dieser Stoffe wird die Verfügbarkeit der Wirkstoffe in der Haarwurzel positiv beeinflusst. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeil der Emulsion. Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Feltsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Erfindungsgemäß besonders geeignete Emulgatoren sind Polyethylenglycolalkylether mit Alkylkettenlängen von 6 bis 30 C-Atomen, bevorzugt von 12 bis 22 C-Atomen und einem Ethoxylierungsgrad von 1 bis 1000, bevorzugt von 1 bis 500 und insbesondere von 1 bis 100.

Insbesondere bevorzugte Polyethylenglycolether im Sinne der Erfindung sind die unter der INCI-Bezeichnung bekannten Stoffe Ceteareth-6, Ceteareth-9, Ceteareth-10, Ceteareth-20, Ceteareth-30, Ceteareth-25, Steareth-10 und Steareth-20.

Weiterhin besonders geeignete Emulgatoren im Sinne der Erfindung sind C₁₂-C₂₂-Fettsäuremono- und - diester von Anlagerungsprodukten von 1 bis 30 Mol Ethylenoxid an Polyole mit 3 bis 6 Kohlenstoffatomen, insbesondere an Glycerin. Insbesondere geeignet sind PEG-20 Glyceryl Stearate, PEG-7 Glyceryl Cocoate, PEG-30 Glyceryl Stearate, PEG-6 Caprylic/Capric Glycerides, Polyglyceryl-3-diisostearate und Glycereth-2-Cocoate.

Bevorzugt sind nichtionogene Emulgatoren mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen. Nichtionogene Emulgatoren mit einem HLB-Wert von 10 bis 16 sind erfindungsgemäß besonders bevorzugt.

Erfindungsgemäß geeignete Dialkylether sind Di-n-alkylether mit insgesamt 5 bis 50 C-Atomen, bevorzugt mit 8 und 40 und insbesondere mit 12 bis 24 C-Atomen, wie beispielsweise Di-n-octylether (Cetiol^{®} OE), Di-n-decylether, Di-n-nonylether, Di-n-undecylether, Di-n-dodecylether, n-Hexyl-n-oetylether, n-Octyl-n-decylether, n-Decyl-n-undecylether, n-Underyl-n-dodecylether und n-Hexyl-n-Undecylether sowie Di-tert-butylether, bi-iso-pentylether. Di-3-ethyldecylether, tert.-Butyl-n-oclylether, iso-Pentyl-n-octylether, 2-Methyl-pentyl-n-octylether und 1,3-Di-(2-ethyl-hexyl)-cyclohexan (Cetiol^{®} S).
Erfindungsgemäß besonders geeignet ist Dicaprylylether.

Bevorzugt verwendete erfindungsgemäße kosmetische Mittel sind dadurch gekennzeichnet, daß sie zusätzlich amphotere(s) Tensid(e), vorzugsweise aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen In der Alkylgruppe,
- N-Kokosalkylaminopropionat,
- Kokosacylaminoethylaminopropionat
- C12 - C18 - Acylsarcosin,
- N-Alkyl-N,N-dimethylammonium-glycinete, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCl-Bezeichnung Cocamidopropyl Betain bekannten Verbindungen,
- der unter der INCl-Bezeichnung Disodium Cocoamphodiacetate bekannten Verbindungen,
enthalten.

Diese sind bevorzugt von 1 bis 15 Gew.-%. vorzugsweise 2,5 bis 12 Gew.-% und insbesondere bevorzugt 5 bis 10 Gew.-%. jeweils bezogen auf das gesamte Mittel, In den erfindungsgemäß verwendeten kosmetischen Mitteln enthalten.

Eine weitere bevorzugte Gruppe von Inhaltsstoffen der erfindungsgemäßen Mittel sind Vitamine, Provitamine oder Vitaminvorstufen. Diese werden nachfolgend beschrieben:
Zur Gruppe der als Vitamin A bezeichneten Substanzen gehören das Retinol (Vitamin A₁) sowie das 3,4-Didehydroretinol (Vitamin A₂). Das β-Carolin.ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Mittel enthalten die Vitamin A-Komponente bevorzugt in Mengen von 0,05-1 Gew.-%, bezogen auf die gesamte Zubereitung.
Zur Vitamin B-Gruppe oder zu dem Vitamin B-Komplex gehören u. a.
   - Vitamin B₁ (Thiamin)
   - Vitamin B₂ (Riboflavin)
   - Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
   - Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05-10 Gew.-%, bezogen auf das gesamte Mittel, enthalten. Mengen von 0,1 - 5 Gew.-% sind besonders bevorzugt.
   - Vitamin B₆ (Pyridoxin sowie Pyridoxamin und Pyridoxal).

Vitamin C (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäß verwendete kosmetische Mittel bevorzugt, die zusätzlich Vitamine und/oder Pro-Vitamine und/oder Vitaminvorstufen enthalten, die vorzugsweise den Gruppen A, B, C, E, F und H zugeordnet werden, wobei bevorzugte Mittel verwendet sind, die die genannten Verbindungen in Mengen von 0.1 bis 5 Gew.-%, vorzugsweise von 0,25 bis 4 Gew.-% und insbesondere von 0,5 bis 2.5 Gew.-%, jeweils bezogen auf das gesamte Mittel, enthalten.

Die erfindungsgemäß verwendeten kosmetischen Mittel können weiterhin alle für solche Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Diese Tenside wurden weiter oben ausführlich beschrieben.

Demgemäß können die erfindungsgemäß verwendeten Mittel beispielsweise zusätzliche Bestandteile enthalten, ausgewählt aus der Gruppe, umfassend Proteinhydrolysate, filmbildende Substanzen, weitere haarwuchsstimullerende Wirkstoffe, nichtionogene Tenside, anionische Tenside, zwitterionische Tenside, ampholytische Tenside, nichtionische Polymere, Pflegestoffe, Verdickungsmittel, Parfümöle, Farbstoffe, Lichtschutzmittel, Antioxidantien, Antischuppenwirkstoffe, Treibmittel, Reduktionsmittel.

Bei den erfindungsgemäß verwendeten Mitteln handelt es sich vorzugsweise um Shampoos, Haarnachspülmittel, Haarwässer, Haargele, Haarkuren, Haarcreme, Haarlotionen, Haarsprays oder Haartinkturen.

Nach der Applikation der Mittel in Form von Haargel, Haarwasser, Haarkur, Haarcreme, Haarlotion, Haarspray und Haartinktur ist es zumeist nicht notwendig das Haar bzw. die Kopfhaut noch einmal auszuspülen oder mit weiteren zusätzlichen Mitteln zu behandeln, um eine positive Wirkung des Mittels zu erreichen. Die Mittel können natürlich ausgespült werden, vorzugsweise sollten sie jedoch auf dem Haar verbleiben.
Bei Shampoos und Haarnachspülmitteln kann das Mittel nach einer Einwirkzeit ausgespült werden. Dieses Ausspülen kann mit reinem Wasser oder einem marktüblichen Shampoo erfolgen. Einwirkzeiten von 10 Sekunden bis 15 Minuten haben sich in den meisten Fällen als ausreichend erwiesen.
Unabhängig von dem genauen Ablauf der Behandlung hat es sich als vorteilhaft erwiesen, die Mitteln bei einer Temperatur von 20 bis 55°C, insbesondere von 35 bis 40°C, anzuwenden.

Erfindungsgemäß von besonderem Interesse sind Haartonics, insbesondere als auf dem Haar verbleibende (leave on) Formulierung. Diese werden vorzugsweise bei Raumtemperatur angewendet, der alkoholische Gehalt liegt bevorzugtermaßen im Bereich von etwa 30 % bis etwa 35 % und der pH-Wert sollte etwa bei pH 7 liegen.

Als Pflegestoff kann beispielsweise ein Silikonöl und/oder ein Silikongum eingesetzt werden. In einer besonderen Ausführungsform der Erfindung enthalten die Mittel mindestens ein Silikonöl und/oder ein Silikongum.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Obwohl die Mittel als Pflegestoff vorzugsweise ein Silikonderivat enthält, ist es auch möglich, dass die Mittel statt oder neben einer Silikonkomponente mindestens einen Pflegestoff einer anderen Verbindungsklasse enthält.

Als Pflegestoff einer anderen Verbindungsklasse können die Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Besonders interessant ist der Einsatz von Seiden-Proteinhydrolysaten. Unter Seide versteht man die Fasern des Kokons des Maulbeer-Seidenspinners (Bombyx mori L.). Die Rohseidenfaser besteht aus einem Doppelfaden Fibroin. Als Kittsubstanz hält Sericin diesen Doppelfaden zusammen. Seide besteht zu 70 - 60 Gew.-% aus Fibroin, 19 - 28 Gew.-% Sericin, 0,5 - 1 Gew.-% aus Fett und 0,5 - 1 Gew.-% aus Farbstoffen und mineralischen Bestandteilen.

Die wesentlichen Bestandteile des Sericin sind mit ca. 46 Gew.-% Hydroxyaminosäuren. Das Sericin besteht aus einer Gruppe von 5 bis 6 Proteinen. Die wesentlichen Aminosäuren des Sericines sind Serin (Ser, 37 Gew.-%), Aspartat (Asp, 26 Gew.-%), Glycin (Gly, 17 Gew.-%), Alanin (Ala), Leucin (Leu) und Tyrosin (Tyr).

Das wasserunlösliche Fibroin ist zu den Skleroproteinen mit langkettiger Molekülstruktur zu zählen. Die Hauptbestandteile des Fibroin sind Glycin (44 Gew.-%), Alanin (26 Gew.-%), und Tyrosin (13 Gew.-%). Ein weiteres wesentliches Strukturmerkmal des Fibroins ist die Hexapeptidsequenz Ser-Gly-Ala-Gly-Ala-Gly.

Technisch ist es auf einfache Art und Weise möglich, die beiden Seidenproteine voneinander zu trennen. So verwundert es nicht, dass sowohl Sericin als auch Fibroin als Rohstoffe zur Verwendung in kosmetischen Produkten jeweils für sich allein bekannt sind. Weiterhin sind Proteinhydrolysate und - derivate auf der Basis der jeweils einzelnen Seidenproteine bekannte Rohstoffe in kosmetischen Mitteln. So wird beispielsweise Sericin als solches seitens der Fa. Pentapharm Ltd. als Handelsprodukt mit der Bezeichnung Sericin Code 303-02 vertrieben. Weitaus häufiger noch wird Fibroin als Proteinhydrolysat mit unterschiedlichen Molekulargewichten im Markt angeboten. Diese Hydrolysate werden insbesondere als "Seidenhydroylsate" vertrieben. So wird beispielsweise unter der Handelsbezeichnung Promois^{®} Silk hydrolysiertes Fibroin mit mittleren Molekulargewichten zwischen 350 und 1000 vertrieben.

Die positiven Eigenschaften der Seidenproteinderivate aus Sericin und Fibroin sind jeweils für sich genommen in der Literatur bekannt. So beschreibt die Verkaufsbroschüre der Fa. Pentapharm die kosmetischen Effekte des Sericines auf der Haut als reizlindernd, hydratisierend und filmbildend. Die Wirkung eines Fibroinderivates wird beispielsweise in der DE 31 39 438 A1 als pflegend und avivierend für das Haar beschrieben. Gemäß DE 102 40 757 A1 lässt sich bei einer gleichzeitigen Verwendung von Sericin und Fibroin bzw. deren Derivaten und/oder Hydrolysaten darüber hinaus eine synergistische Steigerung der positiven Wirkungen der Seidenproteine und deren Derivate erzielen.

Bevorzugt wird daher in den erfindungsgemäß verwendeten Mitteln als Seiden-Proteinhydrolysat ein Wirkstoffkomplex (A) bestehend aus dem Wirkstoff (A1) ausgewählt aus Sericin, Sericinhydrolysaten und/oder deren Derivaten, sowie Mischungen hieraus, und einem Wirkstoff (A2) ausgewählt aus Fibroin, und/oder Fibroinhydrolysaten und/oder deren Derivaten und/oder Mischungen hieraus eingesetzt.

Der Wirkstoffkomplex (A) verbessert signifikant in synergistischer Weise die zuvor dargestellten wesentlichen inneren und äußeren Strukturmerkmale und die Festigkeit sowie die Elastizität von menschlichen Haaren.

Als Wirkstoffe (A1) können im Wirkstoffkomplex (A) verwendet werden:
- natives Sericin,
- hydrolysiertes und/oder weiter derivatisiertes Sericin, wie beispielsweise Handelsprodukte mit den INGI - Bezeichnungen Sericin, Hydrolyzed Sericin, oder Hydrolyzed Silk,
- eine Mischung aus den Aminosäuren Serin, Aspartat und Glycin und/oder deren Methyl-, Propyl-, isoPropyl-, Butyl-, iso-Butylestern, deren Salze wie beispielsweise Hydrochloride, Sulfate, Acetate, Citrate, Tartrate , wobei in dieser Mischung das Serin und/oder dessen Derivate zu 20 bis 60 Gew.%, das Aspartat und/oder dessen Derivate zu 10 - 40 Gew.% und das Glycin und/oder dessen Derivate zu 5 bis 30 Gew.% enthalten sind, mit der Maßgabe, dass sich die Mengen dieser Aminosäuren und/oder deren Derivate vorzugsweise zu 100 Gew. % ergänzen,
- sowie deren Mischungen.

Als Wirkstoffe (A2) können im Wirkstoffkomplex (A) verwendet werden:
- natives, in eine lösliche Form überführtes Fibroin,
- hydrolysiertes und/oder weiter derivatisiertes Fibroin, besonders teilhydrolisiertes Fibroin, welches als Hauptbestandteil die Aminosäuresequenz Ser-Gly-Ala-Gly-Ala-Gly enthält,
- die Aminosäuresequenz Ser-Gly-Ala-Gly-Ala-Gly,
- eine Mischung der Aminosäuren Glycin, Alanin und Tyrosin und/oder deren Methyl-, Propyl-, isoPropyl-, Butyl-, iso-Butylestern, deren Salze wie beispielsweise Hydrochloride, Sulfate, Acetate, Citrate, Tartrate , wobei in dieser Mischung das Glycin und/oder dessen Derivate in Mengen von 20 - 60 Gew.%, das Alanin und dessen Derivate in Mengen von 10 - 40 Gew,% und das Tyrosin und dessen Derivate in Mengen von 0 bis 25 Gew.% enthalten sind, mit der Maßgabe, dass sich die Mengen dieser Aminosäuren und/oder deren Derivate vorzugsweise zu 100 Gew.-% ergänzen,
- sowie deren Mischungen.

Besonders gute pflegende Eigenschaften können erzielt werden, wenn eine der beiden Wirkstoffkomponenten des Wirkstoffkomplexes (A) in der nativen oder allenfalls löslich gemachten Form verwendet wird. Es ist auch möglich, eine Mischung aus mehreren Wirkstoffen (A1) und/oder (A2) einzusetzen.

Es kann bevorzugt sein, dass die beiden Wirkstoffe (A1) und (A2) im Verhältnis von 10:90 bis 70:30, insbesondere 15:85 bis 50:50 und ganz besonders 20:80 bis 40:60, bezogen auf deren jeweilige Gehalte an aktiver Wirksubstanz in den erfindungsgemäß verwendeten Mitteln eingesetzt werden.

Die Derivate der Hydrolysate von Sericin und Fibroin umfassen sowohl anionische als auch kationisierte Proteinhydrolysate. Die Proteinhydrolysate von Sericin und Fibroin sowie die daraus hergestellten Derivate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche Proteinhydrolysate von Sericin und Fibroin und/oder deren Derivate, deren zugrunde liegender Proteinanteil ein Molekulargewicht von 100 bis zu 25000 Dalton, bevorzugt 250 bis 10000 Dalton aufweist. Weiterhin sind unter kationischen Proteinhydrolysaten von Sericin und Fibroin auch quaternierte Aminosäuren und deren Gemische zu verstehen. Die Quaternisierung der Proteinhydrolysate oder der Aminosäuren wird häufig mittels quartärer Ammoniumsalze wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäß einsetzbaren kationischen Proteinhydrolysate und - derivate seien die unter den INCl - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Silk, Cocodimonium Hydroxypropyl Silk Amino Acids, Hydroxyproypltrimonium Hydrolyzed Silk, Lauryldimonium Hydroxypropyl Hydrolyzed Silk, Steardimonium Hydroxypropyl Hydrolyzed Silk, Quaternium-79 Hydrolyzed Silk. Als typische Beispiele für die erfindungsgemäßen anionischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Potassium Cocoyl Hydrolyzed Silk, Sodium Lauroyl Hydrolyzed Silk oder Sodium Stearoyl Hydrolyzed Silk. Letztlich seien noch als typische Beispiele für die erfindungsgemäß einsetzbaren Derivate aus Sericin und Fibroin die unter den INCI - Bezeichnungen im Handel erhältlichen Produkte genannt: Ethyl Ester of Hydrolyzed Silk und Hydrolyzed Silk PG-Propyl Methylsilanediol. Weiterhin erfindungsgemäß verwendbar, wenngleich nicht unbedingt bevorzugt sind die im Handel erhältlichen Produkte mit den INCI - Bezeichnungen Palmitoyl Oligopeptide, Palmitoyl Pentapaptide-3, Palmitoyl Pentapeptide-2, Acetyl Hexapeptide-1, Acetyl Hexapeptide-3, Copper Tripeptide-1, Hexapeptide-1, Hexapeptide-2, MEA-Hydrolyzed Silk.

Die Wirkung des Wirkstoffkomplexes (A) kann durch die Zugabe von Fettstoffen weiter gesteigert werden. Unter Fettstoffen sind zu verstehen Fettsäuren, Fettalkohole, natürliche und synthetische Wachse, welche sowohl in fester Form als auch flüssig in wässriger Dispersion vorliegen können, und natürliche und synthetische kosmetische Ölkomponenten zu verstehen.

Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in den erfindungsgemäß verwendeten Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als filmbildende Substanzen können einige pflegende Polymere auch filmbildende und/oder festigende Eigenschaften aufweisen, und daher auch als filmbildende und/oder festigende Polymere eingesetzt werden können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I). 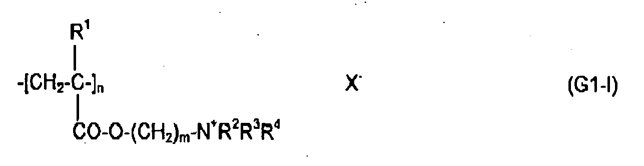 in der R¹= -H oder -CH₃ ist. R², R³ und R⁴ unabhängig voneinander ausgewählt sind aus C₁₋₄-Alkyl-,-Alkenyl- oder -Hydroxyalkylgruppen, m =1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliche Gegenionen X kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezelchnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen, Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCl-Bezeichnung: Mineral Oil) und Tridecyl-polyoxypropylen-polyoxyethylen-ether (INCl-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCl-Bezeichnung: Propylene Glycol Dicaprylate/Dicaprate) und Tridecyl-polyoxypropylen-polyoxyethylen-ether (INCl-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester, Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Mathacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im
- Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silikon, das auch als Amodimethicone bezeichnet wird), . SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und - methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugt eingesetzte kationische Polymere sind kationische Cellulose-Derivate und Chitosan und dessen Derivate, Insbesondere die Handelsprodukte Polymer^{®}JR 400, Hydagen^{®} HCMF und Kytamer^{®} PC. kationische Guar-Derivate, kationische Honig-Derivate, insbesondere das Handelsprodukt Honeyquat^{®} 50, kationische Alkylpolyglycodside und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCl - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und Im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Bevorzugt eingesetzte amphotere Polymere sind solche,Polymerisate, die sich im wesentlichen zusammensetzen aus
(a) Monomeren mit quartären Ammoniumgruppen der allgemeinen Formel (II).

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-lon ist; Acrylamidopropyltrimethyl-ammoniumchlorid ist ein besonders bevorzugtes Monomeres (a). Als Monomeres (b) für die genannten Polymerisate wird bevorzugt Acrylsäure verwendet.

Die erfindungsgemäß verwendeten Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

In einer besonders bevorzugten Ausführungsform enthalten die erfindungsgemäß verwendeten Mittel weitere haarwuchsstimulierende Wirkstoffe.

Geeignet sind alle bekannten haarwuchsstimuliernden Wirkstoffe, beispielsweise Minoxidil (6-Piperidino-2,4-pyrimidindiamin-3-oxid), Aminexil (Diaminopyrimidinoxid), Finasterid (N-tert-Butyl-3-oxo-4-aza-5α-androst-1-en-17β-carboxamid), Carnitintartrat und 5-α-Reduktaseinhibitoren.

Als 5-α-Reduktaseinhibitoren sind insbesondere geeignet die funktionellen C₂-C₁₂-Carbonsäuren und deren physiologisch verträglichen Metallsalze, insbesondere 10-Hydroxydecansäure, 10-Hydroxydecensäure und ihren Derivate, Derivate von C₃-C₉-Polyolen, Phenolderivate, Pflanzenextrakte, Riechstoffe, Flavonoide, Isoflavonoide, 6,7-disubstituierte 2,2-Dialkylchromane oder -chromene, Aluminiumchlorohydrat, 2-Phenylethanol, Etidronsäure, 7-Acetyl-1,1,3,4,4,6-hexamethyltetralin, Tropolonderivate, Ester der Schwefelsäure mit alkoxylierten C₈-C₁₈-Fettalkoholen und deren physiologisch verträgliche Metallsalze, Ester der Phosphorsäure und der Triphosphorsäure mit ein- bis sechswertigen Hydroxyverbindungen, Kieselsäureester, aus marinen Organismen isolierbare mycosporin-ähnliche Aminosäuren (MAA) sowie quaternäre Siliconverbindungen. Unter Derivaten sind dabei insbesondere deren Salze, Ester und Amide zu verstehen.

In einer bevorzugten Ausführungsform können die erfindungsgemäß verwendeten Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 90 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäß verwendeten Mitteln Mischungen aus mehreren, insbesondere aus zwei, verschiedenen Pflanzenextrakten einzusetzen.
Weiterhin können die erfindungsgemäß verwendeten Mittel als Pflegestoff eine Reihe von Carbonsäuren enthalten.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren Im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß einsetzbaren Carbonsäuren können selbstverständlich entlang der Kohlenstoffkette oder des Ringgerüstes substituiert sein. Zu den Substituenten der erfindungsgemäß einsetzbaren Carbonsäuren sind beispielsweise zu zählen C₁-C₈-Alkyl-, C₂-C₈-Alkenyl-, Aryl-, Aralkyl- und Aralkenyl-, Hydroxymethyl-, C₂-C₆-Hydroxyalkyl-,C₂-C₈-Hydroxyalkenyl-, Aminomethyl-, C₂-C₈-Aminoalkyl-, Cyano-, Formyl-, Oxo-, Thioxo-, Hydroxy-, Mercapto-, Amino-, Carboxy- oder Iminogruppen. Bevorzugte Substituenten sind C₁-C₆-Alkyl-, Hydroxymethyl-, Hydroxy-, Amino- und Carboxygruppen. Besonders bevorzugt sind Substituenten in α-Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäß einsetzbare Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 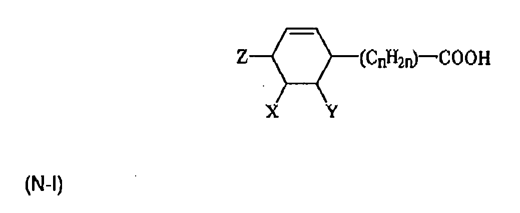 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt. So ist beispielweise US-A 3.753,968 ein Herstellungsverfahren zu entnehmen.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuss vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbindung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt 2-Pyrrotidinon-5-carbonsäuro und deren Derivate als Carbonsäure einzusetzen. Besonders bevorzugt sind die Natrium-, Kalium-, Calclum-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäß verwendeten Mitteln betragen vorzugsweise 0,05 bis 10 Gew.%, bezogen auf die gesamte Anwendungszubereitung, besonders bevorzugt 0,1 bis 5 Gew.%, und insbesondere bevorzugt 0,1 bis 3 Gew.%.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, dass neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8-22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Weiterhin können die erfindungsgemäß verwendeten Mittel als Pflegestoff Ölkörper enthalten.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert-Butyl-n-octylether, iso-Pentyl-n-octylether und 2-Methyl-pentyl-n-ootylether. Die als Handelsprodukte erhältlichen Verbindungen 1,3-Di-(2-ethyl-hexyl)-cyclohexan (Cetiol^{®} S) und Di-n-octylether (Cetiol^{®}OE) können bevorzugt sein.
- Esteröle: Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure. Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), lsononansäure-C16-18-alkylester (Cetiol^{®} SN), 2-Ethylhexylpalmitat (Cegesoft^{®} 24), Stearinsäute-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl lsononanoate (Cetiol^{®} SN), Ölsäuredecylester (Celiol^{®} V).
- Dicarbonsäureester wie Di-n-butyladipat, Di-(2-ethylhexyl)-adipat, Di-(2-ethylhexyl)-succinat und Diisotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC).
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 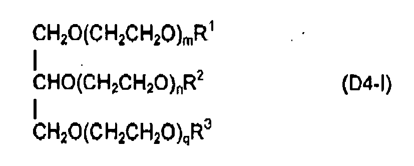 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.%, und insbesondere 0,1 -15 Gew.-%.

Das erfindungsgemäß verwendete Mittel kann überdies ein Enzym als Pflegestoff enthalten. Erfindungsgemäß besonders bevorzugte Enzyme sind ausgewählt aus einer Gruppe, die gebildet wird aus Proteasen, Lipasen, Transglutaminase, Oxidasen und Peroxidasen.

Die erfindungsgemäß verwendeten Mittel enthalten die Dialkylether bevorzugt in Mengen von 0,01 - 50 Gew.-%, bevorzugt in Mengen von 0,05 bis 20 Gew.-% und insbesondere in Mengen von 0,1 - 15 Gew.-%, bezogen auf das gesamte Mittel.

Es kann weiterhin erforderlich sein, dass die Mittel weiterhin mindestens ein Verdickungsmittel enthalten. Dies trifft insbesondere dann zu, wenn es sich bei dem Mittel um ein Gel oder eine Paste handelt.

Geeignete Verdickungsmittel sind beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcallulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate. Auch die Polymere, die als mögliche Pflegestoffe beschrieben wurden oder im Folgenden als filmbildende und/oder festigende Polymere aufgezählt werden, können verdickende Eigenschaften aufweisen, und können gegebenenfalls als Verdickungsmittel Verwendung finden.

Die Mittel können neben den genannten Komponenten weiterhin alle für entsprechende kosmetische Mittel bekannten Wirk-, Zusatz- und Hilfsstoffe enthalten. Dies sind beispielsweise
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkyllmidazolinium-methosulrat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, N₂O, Dimethylether, CO₂ und Luft.
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der Mittel kann in allen für kosmetische Mittel üblichen Formen erfolgen, beispielsweise in Form von Lösungen, die als Haarwasser oder Pump- oder Aerosolspray auf das Haar aufgebracht werden können, in Form von Emulsionen, Wachsen, Gelen oder auch tensidhaltigen schäumenden Lösungen oder anderen Zubereitungen, die für die Anwendung auf dem Haar geeignet sind.

In einer bevorzugten Ausführungsform handelt es sich bei den erfindungsgemäß verwendeten Mittel um Haarbehandlungsmittel, insbesondere Shampoo, Haarnachspülmittel, Haartonic, Haargel, Haarwasser, Haarkur, Haarcreme, Haarlotion, Haarspray und Haartinktur.

Die Mittel können beispielsweise als Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaum aerosole formuliert vorliegen. Die Mittel können als Einkammersystem oder als Zweikammersystem konfektioniert werden.

Sofern es sich bei den verwendeten Mitteln um ein Aerosolprodukt handelt, enthält dieses zwingend ein Treibmittel.

Erfindungsgemäß geeignete Treibmittel sind beispielsweise N20, Dimethylether, CO2, Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen.

Bevorzugt werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, Insbesondere von 3 bis 15 Gew.-% sind besonders bevorzugt. Aerosolsprays enthalten generell größere Mengen an Treibmittel. Bevorzugt wird das Treibmittel in diesem Fall In einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Wenngleich die zuvor genannten Haarbehandlungsmittel erfindungsgemäß bevorzugt sind, kann es sich bei den Mitteln auch um andere Haarbehandlungsmittel, wie z.B. Haarfärbemittel und Wellmittel, handeln. Diese Mittel enthalten dann gegebenenfalls die bekannten direktziehenden Farbstoffe, Vorläufer für Oxidationsfarbstoffe (Entwickler- und Kupplerkomponenten) und Oxidationsmittel bzw. Reduktionsmittel. Vorteilhafterweise können die Mittel so das Haar vor der Beanspruchung bei der Färbung schützen, das Haarfollikel aktivieren und gleichzeitig Hautirritationen durch die Well- bzw. Haarfärbemitteln vermindern bzw. lindern.

Die nachfolgenden Beispiele sollen den Gegenstand der vorliegenden Erfindung erläutern ohne ihn in irgendeiner Weise zu beschränken.

### Beispiel 1: Nachweis der Freisetzung des Keratinocyte Growth Factors (KGF) unter Verwendung dermaler Papillenzellen

Der Keratinozyten Wachstumsfaktor Factor (KGF) Ist ein äußerst wichtiger Regulator des Haars. Bei einer potentiell wachstumsfördernden und das Haar stärkenden Aktivsubstanz kann von einer Steigerung der ins Medium ausgeschütteten Faktoren ausgegangen werden. Die Ausschüttung von KGF kann mit Hilfe von kommerziell erhältlichen ELISA-Kits quantifiziert werden. Dazu wurden die dermalen Papillenzellen über 24 Stunden mit Theophyllin inkubiert und die Konzentration des Wachstumsfaktors im Medium in beschriebener Weise quantifiziert.

**Tabelle 1: Relative Ausschüttung von KGF in [%] nach Behandlung der dermalen Papillenzellen im Vergleich zur unbehandelten Kontrollen.**

| | KGF | |
|---|---|---|
| | Mittelwert | Standardabweichung |
| Kontrolle | 100 | 12,4 |
| Theophyllin 100µM | 161,3 | 10,9 |
| Theophyllin 10µM | 134,8 | 16,5 |
| Theophyllin 1µM | 121,4 | 7,7 |

Die Behandlung mit Theophyllin führte bei dermalen Papillenzellen zu einer signifikant erhöhten Freisetzung von KGF.

KGF wird von der dermalen Papille, dem Steuerzentrum des Haarfollikels, ausgeschüttet, und reguliert die Proliferation von Haarschaftkeratinozyten. Diese führen durch Synthese typischer Keratine des Haares zur Ausprägung des Haarschaftes. Durch eine verbesserte Stimulation der Haarschaftkeratinozyten durch Theophyllin kann somit die Struktur des Haares positiv beeinflusst werden. Zusätzlich fördert KGF auch die Proliferation der Haarmatrix-Keratinozyten. Dadurch verdickt sich der Haarbulbus und das Haar wächst dicker aus der Wurzel nach.

### Beispiel 2: Nachweis der Freisetzung von Wachstumsfaktor unter Verwendung von organotypischen Haarfollikelzellkulturen

Hepatocyte Growth Factor (HGF) und Keratinocyte Growth Factor (KGF) sind wichtige Wachstumsfaktoren, die von der dermalen Papille ausgeschüttet werden, um die Proliferation der Haarschaftkeratinozyten, die für die Synthese der Haarkeratine verantwortlich sind, zu steuern. Sie sind außerdem charakteristische Marker für die Anagenphase, in der auch die Keratinsynthese maximal ist. Darüber hinaus ist zu beachten, dass bei der Haaralterung das Proliferationsvermögen der Haarfollikelzellen abnimmt. Die Ausschüttung von HGF sowie KGF kann mit Hilfe von kommerziell erhältlichen ELISA-Kits quantifiziert werden.

Dazu werden organotypische Haarfollikelzellkulturn über 72h mit Theophyllin inkubiert und die Konzentration der Wachstumsfaktoren im Medium in beschriebener Weise bestimmt.

**Tabelle 2: Relative Ausschüttung von HGF und KGF in [%] nach Behandlung des organotypischen Haarfollikelzellkulturen im Vergleich zu unbehandelten Kontrollen.**

| | Konzentration | KGF | HGF |
|---|---|---|---|
| | [µM] | Mittelwert | Mittelwert |
| Unbehandelte Probe | | 100 (35) | 100 (14) |
| Theophyllin | 500 | 144 (11) | 144 (16) |

Die Behandlung mit Theophyllin (500 µM) führte unter Verwendung von organotypischen Haarfollikelzellkulturen zu einer tendenziell erhöhten Freisetzung von KGF. Die hier nachgewiesene erhöhte Freisetzung beruht auf der gesteigerten Synthese der dermalen Papillenzellen wie In Beispiel 1 gezeigt. Überraschenderweise wurde hierbei auch eine erhöhte Freisetzung des Hepatozyten Wachstumsfaktor (HGF) nachgewiesen. Dies beruht auf der interzellulären Kommunikation zwischen den dermalen Papillenzellen und den dermalen Fibroblasten, die ebenfalls im organotypischen Modell enthalten sind. HGF steuert beispielsweise die Zellproliferation in der Haarmatrix. Durch die gesteigerte Proliferation kann die Haarmatrix verdickt und so potentiell das Volumen der Haarfaser beeinflusst werden.

### Beispiel 3: Nachweis der Aktivierung der Keratinsynthese an kultivierten Haarkeratinozyten

Die Festigkeit der Haare und die Haarstruktur sind im Wesentlichen von der Zusammensetzung spezieller haarspezifischer Strukturproteine abhängig, den Haarkeratinen. Durch die Veränderung der Zusammensetzung dieser spezifischen Proteine wird auf biologischer Ebene Einfluss auf die Haarstruktur genommen. Darüber hinaus nehmen im Alter bestimmte Haarkeratine ab, was zu strukturellen Veränderungen im Altershaar führt, das Haar wird beispielsweise brüchig.

Zur Bestimmung des Einflusses von Theophyllin auf die Expression von Haarkeratinen wurde die differentielle Genexpression mittels quantitatitiver RT-PCR bestimmt. Nach Anzucht der Keratinozyten der äusseren Wurzelscheide (ORS-Keratinozyten) wurden diese für 3, 6 oder 24 h mit Theophyllin in Konzentration von 1.10 und 100 µM inkubiert.

Zur Durchführung der PCR wird zunächst mit Hilfe des RNeasy Mini Kits der Fa. Qiagen die RNA aus ORS-Keratinozyten isoliert und mittels reverser Transkription in cDNA umgeschrieben. Bei der anschließenden PCR Reaktion, die mit Hilfe genspezifischer Primer für die jeweiligen haarrelevanten Gene durchgeführt wird und die der Amplifikation der gesuchten Genabschnitte dient, wird die Bildung der PCR-Produkte online über ein Fluoreszenzsignal detektiert. Das Fluoreszenzsignal ist dabei proportional zur Menge des gebildeten PCR-Produktes. Je stärker die Expression eines bestimmten Gens Ist, desto größer Ist die Menge an gebildetem PCR-Produkt und um so höher ist das Fluoreszenzsignal.
Zur Quantifizierung der Genexpression wird die unbehandelte Kontrolle gleich 1 gesetzt und die Expression der zu bestimmenden Gene darauf bezogen (x-fache Expression). Dabei werden Werte, die größer/gleich der 2fachen Expression oder kleiner/gleich der 0.5fachen Expression der unbehandelten Kontrolle sind als signifikant differentiell exprimiert eingestuft. Werte, die größer/gleich der 1,6fachen Expression oder kieiner/gleich der 0.66fachen Expression der unbehandelten Kontrolle sind als tendenziell differentiell exprimiert eingestuft. Die Ergebnisse sind in Tabelle 3 zusammengefasst.

**Tabelle 3: Haarkeratinexpression nach Behandlung von ORS-Keratinozyten in Zellkulturen im Vergleich zur unbehandelten Kontrolle.**

| | | hha3 | hha4 | hhb6 | hha1 | hha6 |
|---|---|---|---|---|---|---|
| | | Mittelwert | Mittelwert | Mittelwert | Mittelwert | Mittelwert |
| 3 h | Unbehandelte Probe | 1,00 | 1,00 | 1,00 | 1,00 | 1,00 |
| | Theophyllin 1 µM | 0,86 | 0,83 | 1,09 | 0,73 | 0,45 |
| | Theophyllin 10 µM | 1,47 | 1,40 | 1,12 | 1,18 | 0,59 |
| | Theophyllin 100 µM | 2,47 | 2,41 | 1,78 | 1,38 | 0,28 |
| 6 h | Unbehandelte Probe | 1,00 | 1,00 | 1,00 . | 1,00 | 1,00 |
| | Theophyllin 1 µM | 0,80 | 0,81 | 0,80 | 0,90 | 1,12 |
| | Theophyllin 10 µM | 0,87 | 0,88 | 0,76 | 0,92 | 0,42 |
| | Theophyllin 100 µM | 1,25 | 1,31 | 1,23 | 1,22 | 0,57 |
| 24 h | Unbehandelte Probe | 1,00 | 1,00 | 1,00 | 1,00 | 1,00 |
| | Theophyllin 1 µM | 1,20 | 1,27 | 1,34 | 1,30 | 3,19 |
| | Theophyllin 10 µM | 1,41 | 1,48 | 1,28 | 1,40 | 2,14 |
| | Theophyllin 100 µM | 2,75 | 2,65 | 1,98 | 2,49 | 1,61 |

Die Behandlung mit Theophyllin führt in den Haarkeratinozyten der äußeren Wurzelscheide (,outer root sheath Keratinocytes") zu einer Konzentration- und Zeit-abhängigen Aktivierung der hier analysierten Haarkeratine.

### Beispiel 4: Nachweis der Aktivierung der Keratinsynthese an organotpischen Haarfollikeizellkulturen

Die Aktivierung der Keratinsynthese nach Applikation von Theophyllin wurde zusätzlich mit Hilfe organotypischer Haarfollikelzelikulturen untersucht, in denen verschiedene Zellen des Haarfollikels miteinander interagieren können. Die Untersuchungen erfolgten mit dem in Beispiel 3 erläuterten Verfahren der quantitativen Reverse Transkription Polymerase Kettenreaktion. Die Ergebnisse sind in Tabelle 4 zusammengefasst.

**Tabelle 4: Haarkeratinexpression nach Behandlung mit Theophyllin in organotpischen Haarfollikelzellkulturen im Vergleich zur unbehandelten Kontrolle.**

| | | hha3 | hha4 | hhb6 |
|---|---|---|---|---|
| | | Mittelwert | Mittelwert | Mittelwert |
| 6 h | Unbehandelte Probe | 1,00 | 1,00 | 1,00 |
| | Theophyllin 10 µM | 0,76 | 0,91 | 1,28 |
| | Theophyllin 100 µM | 0,76 | 1,56 | 2,06 |
| 24 h | Unbehandelt | 1,00 | 1,00 | 1,00 |
| | Theophyllin 10 µM | 2,27 | 2,08 | 1,81 |
| | Theophyllin 100 µM | 3,00 | 1,88 | 1,68 |

Die Behandlung mit Theophyllin führt in den haarspezifischen Keratinozyten der äußeren Wurzelscheide ("outer root sheath keratinocytes") zu einer Konzentration- und Zeit-abhangigen Aktivierung der hier analysierten Haarkeratine.

## Patentansprüche

1. Verwendung von Purin und/oder Purinderivat(en) in kosmetischen Mitteln zur Regulation der Haarkeratine und/oder Verhinderung von Altershaar.

2. Verwendung nach Anspruch 1, **dadurch gekennzeichnet, dass** Purin und/oder Purinderivat(e) der allgemeinen Formel (I) verwendet wird: 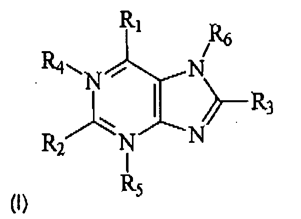 In der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = H)
- Theophyllin (R¹ = R² = OH, R³ = H, R⁴ = CH₃, R⁵ = CH₃, R⁶ = H)

3. Verwendung nach einem der vorhergehenden Ansprüche, **dadurch gekennzeichnet, dass** die Mittel 0,0005 bis 5 Gew.-% Purin und/oder Purinderivat(e) enthalten.

4. Verwendung nach einem der vorhergehenden Ansprüche, **dadurch gekennzeichnet, dass** die Mittel 0,001 bis 2,5 Gew.-%, vorzugsweise 0,0025 bis 1 Gew.-%, besonders bevorzugt 0,005 bis 0,5 Gew.-% und insbesondere bevorzugt 0,01 bis 0,1 Gew.-% Purin(e) und/oder Purinderivat(e) enthalten.

5. Verwendung nach einem der vorhergehenden Ansprüche, **dadurch gekennzeichnet, dass** die kosmetischen Mitteln Tensid(e), vorzugsweise anionische, kationische, zwitterionische, ampholytische und/oder nichtionische Tenside enthalten.

6. Verwendung nach einem der vorhergehenden Ansprüche, **dadurch gekennzeichnet, dass** die kosmetischen Mitteln 1 bis 15 Gew.-%, vorzugsweise 2,5 bis 12 Gew.-% und insbesondere bevorzugt 5 bis 10 Gew.-%, jeweils bezogen auf das gesamte Mittel, amphotere(s) Tensid(e), vorzugsweise aus den Gruppen der
- N-Alkylglycine,
- N-Alkylpropionsäuren,
- N-Alkylaminobuttersäuten,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C12 - C18 - Acylsarcosin,
- N-Alkyl-N,N-dimethylammonium-glycinate, beispielsweise Kokosalkyl-dimethylammoniumglycinat,
- N-Acyl-amlnopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen,
enthalten.

7. Verwendung nach einem der vorhergehenden Ansprüche, **dadurch gekennzeichnet, dass** die kosmetischen Mitteln zusätzlich Vitamine und/oder Pro-Vitamine und/oder Vitaminvorstufen enthalten, die vorzugsweise den Gruppen A, B, C, E, F und H zugeordnet werden, wobei bevorzugte Mittel die genannten Verbindungen in Mengen von 0,1 bis 5 Gew.-%, vorzugsweise von 0,25 bis 4 Gew.-% und insbesondere von 0,5 bis 2,5 Gew.-%, jeweils bezogen auf das gesamte Mittel.

8. Verwendung nach einem der vorhergehenden Ansprüche, **dadurch gekennzeichnet, dass** das Purinderivat Theophyllin ist.

9. Verwendung nach einem der vorhergehenden Ansprüche, **dadurch gekennzeichnet, dass** die kosmetischen Mitteln zusätzliche Bestandteile enthalten, ausgewählt aus Proteinhydrolysate, filmbildende Substanzen, weitere haarwuchsstimulierende Wirkstoffe, nichtionogene Tenside, anionische Tenside, zwitterionische Tenside, ampholytische Tenside, nichtionische Polymere, Verdickungsmittel, Parfümöle, Farbstoffe, Lichtschutzmittel, Antioxidantien, Antischuppenwirkstoffe, Treibmittel, Reduktionsmittel.

10. Verwendung nach einem der vorhergehenden Ansprüche, **dadurch gekennzeichnet, dass** die kosmetischen Mitteln ein Haarbehandlungsmittel, insbesondere Shampoo, Haarnachspülmittel, Haargel, Haartonic, Haarwasser, Haarkur, Haarcreme, Haarlotion, Haarspray und Haartinktur ist.
